# EUROPEAN PATENT SPECIFICATION

(11) **EP 2 790 499 B1**
(45) Date of publication and mention of the grant of the patent: **06.06.2018**
(21) Application number: 12812910.3
(22) Date of filing: 13.12.2012
(51) Int. Cl.: C12N 5/073, C12N 5/075

(54) **CULTURE MEDIA FOR TREATING FEMALE GAMETES AND EMBRYOS IN MEDICALLY ASSISTED REPRODUCTION TECHNIQUES**
KULTURMEDIEN ZUR BEHANDLUNG WEIBLICHER GAMETEN UND EMBRYONEN BEI MEDIZINISCH UNTERSTÜTZTEN REPRODUKTIONSVERFAHREN
MILIEUX DE CULTURE POUR LE TRAITEMENT DE GAMÈTES FEMELLES ET D'EMBRYONS DANS DES TECHNIQUES DE PROCRÉATION MÉDICALE ASSISTÉE

(30) Priority: 13.12.2011 IT FI20110271
(43) Date of publication of application: 22.10.2014
(73) Proprietor: LO. LI. Pharma S.r.l., 00156 Roma (IT)
(72) Inventor: UNFER, Vittorio, 00155 Roma (IT)
(74) Representative: Brighenti, Livio
(86) International application number: PCT/EP2012/075355
(87) International publication number: WO 2013/087755

(56) References cited:
- EP-A1- 2 039 758
- WO-A1-00/32140
- WO-A2-2005/108555
- KIM MIRAN ET AL: "Comparison of in vitro maturation media of immature oocytes: the effectiveness of blastocyst culture media.", FERTILITY AND STERILITY FEB 2011 LNKD- PUBMED:21094941, vol. 95, no. 2, February 2011 (2011-02), pages 554-557, XP002674238, ISSN: 1556-5653
- CHIU TONY TAK YU ET AL: "Effects of myo-inositol on the in-vitro maturation and subsequent development of mouse oocytes", HUMAN REPRODUCTION, OXFORD UNIVERSITY PRESS, GB, vol. 18, no. 2, 1 February 2003 (2003-02-01), pages 408-416, XP008098856, ISSN: 0268-1161, DOI: 10.1093/HUMREP/DEG113
- HASHIMOTO SHU ET AL: "Medium without ammonium accumulation supports the developmental competence of human embryos.", THE JOURNAL OF REPRODUCTION AND DEVELOPMENT OCT 2008 LNKD- PUBMED:18580042, vol. 54, no. 5, October 2008 (2008-10), pages 370-374, XP002674239, ISSN: 0916-8818

## Description

### Field of the invention

The present invention relates to the field of culture media, in particular for treating oocytes and embryos in assisted reproduction.

### State of the art

It is estimated that infertility affects around 80 million people in the world and that at least one in ten couples is affected by primary infertility (childless) or secondary infertility (with children).

The causes of infertility are attributable to both disorders and diseases of the male or female reproductive system, exacerbated if they are present in both partners, and to environmental factors and social life styles, which affect reproductive health at different levels.

While from a biological point of view, a woman's most fertile period is at around 20 years of age, education and consolidating a career, often result in women attempting to get pregnant at around 33-35 years of age, an age at which reproduction potential is already in sharp decline. Thus, even in the absence of specific reproductive disorders, the mere time shift in attempting to get pregnant is often sufficient to exacerbate those causes of subfertility that may already be present within the couple.

In the last 30 years, research in the field of Reproductive Biology has provided fundamental knowledge for understanding of the mechanisms that are at the basis of gamete maturation, fertilisation and embryo development. This knowledge has provided the basis for the development of the biotechnologies that are currently applied in medically assisted procreation.

The efficiency of the medically assisted procreation techniques is closely related to the number and to the quality of the zygotes obtained, which depends on the number and on the quality of the oocytes collected and on the number (motility and morphology) of spermatozoa collected; nowadays the percentage success rate of MAP techniques is around 30%.

A possible role of natural substances, also including inositol, has been hypothesised in gamete maturation, however studies carried out on oocytes of bovine origin have shown that myo-inositol had no effect on the development rate of the blastocysts of embryos cultured in a medium supplemented with said substances (Momozawa et al. 2011 Journal of reproduction and development).

In particular, the medium used in these studies was not a medium specifically developed for human MAP techniques and already contained myo-inositol.

### Summary of the invention

The invention relates to the culture media for use in the treatment of oocytes and embryos in medically assisted reproduction techniques.

### Detailed description of the invention

The present invention allows the aforementioned needs to be met thanks to culture media supplemented with inositol, for treating oocytes and embryos in medically assisted reproduction techniques.

Culture medium according to this invention means the normal culture media used for the *in vivo* treatment of oocytes and embryos, which, in addition to the usual components, also however contains inositol.

In particular, according to the invention, a culture medium suitable for such purposes can comprise inositol, sodium chloride, potassium chloride, magnesium sulfate, potassium sulfate, calcium lactate, sodium bicarbonate, glucose, sodium pyruvate, sodium lactate, taurine, alanine - glutamine, EDTA, gentamicin, phenol red, human serum albumin, HEPES.

According to the invention, inositol means any one of the nine different stereoisomers thereof: myo-inositol, D-chiro-inositol; L-chiro-inositol, scyllo-inositol, muco-inositol, neo-inositol, allo-inositol, epi-inositol and cis-inositol or mixtures thereof; myo-inositol being preferred.

The final concentration of inositol is preferably between 0.5 mg/ml and 50 mg/ml (preferably 1 mg/ml and 20 mg/ml).

The inositol can be added to the common culture media alone or in combination with other substances, in the form or powder or solution.

According to one particular embodiment of the invention, in addition to the inositol, melatonin and glutathione can also be added to the traditional culture medium; if present, these are normally in a quantity between 10 - 300 pg/ml and 0.23 - 23.22 mg/ml.

According to a particular embodiment of the invention, a culture medium consists as follows:

| Component | Amount |
|---|---|
| Myo-inositol | 1-20 mg/ml |
| Sodium chloride | 5-7mg/ml |
| Potassium chloride | 0.3-0.5 mg/ml |
| Magnesium sulfate | 0.02-0.03 mg/ml |
| Potassium sulfate | 0.002-0.01 mg/ml |
| Calcium lactate | 0.05-0.07 mg/ml |
| Sodium bicarbonate | 0.3-0.4 mg/ml |
| HEPES | 4.5-5.5 mg/ml |
| Glucose | 0.45-0.55 mg/ml |
| Sodium pyruvate | 0.2-0.4 mg/ml |
| Sodium lactate | 0.00045-0.00055 mM |
| Taurine | 0.008-0.01 mg/ml |
| Alanyl-Glutamine | 0.2-0.3 mg/ml |
| EDTA | 0.002-0.003 mg/ml |
| Gentamicin | 0.004-0.005 mg/ml |
| Phenol red | 2.7-3.3 mg/L |
| Human serum albumin | 4.5-5.5 mg/L |

### Example 1

133 mg of myo-inositol were dissolved in 1 ml of saline solution. The solution thus prepared was diluted 66 times to the culture medium so as to obtain a new culture medium having a myo-inositol concentration of 2 mg/ml.

As recorded in greater detail in the below Example, it was surprisingly found that using the culture medium as prepared above, a greater number of oocytes were fecunded and gave origin to an embryo if the fecundation procedure is carried out in a medium with added inositol (fecundation rate).

In addition, a greater number of embryos cultured in a medium with added inositol continued in the various stages of embryo development. In particular, after 96 hr of fecundation, a greater number of embryos had reached blastocyst or expanded blastocyst status, also indicating a velocity in the embryo development process similar to the physiological velocity. Furthermore, following the transfer of the embryos cultured in the presence of inositol into surrogate mothers, it was observed that a greater number of embryos were able to implant themselves in the uterus as evidenced by the number of births.

### Example 2

160 oocytes from the C57BI6 mouse were equally divided into two groups.

The oocytes in the first group were fecunded and the resulting embryos cultured using the normal techniques of the prior art, in an *in vitro* fecundation medium of the type used in assisted fecundation clinics but with added myo-inositol at a final concentration of 2 mg/ml; on the other hand, the oocytes of the second group were not fecunded, and the resulting embryos were cultured *in vitro* in the same fecundation culture but without myo-inositol.

The results obtained have shown that the fecundation rate is greater in the medium with added myo-inositol, increasing from 40 to 65%.

Furthermore, after 96 hr of culture only 5% of the embryos cultured in the presence of myo-inositol saw arrested development at the 1 cell stage (embryos that will never develop) versus 19% of the control embryos (embryos cultured in a medium without myo-inositol).

In the same way, 50% of the embryos cultured in myo-inositol reach the expanded blastocyst stage versus 31% of those cultivated in a control medium.

On transferring the same number of embryos into surrogate mothers, it was then observed that the group of embryos cultured in myo-inositol resulted in a number of births around 30% higher than the control group.

## Claims

1. The culture media for use in the treatment of oocytes and embryos in medically assisted reproduction techniques, wherein these culture media are supplemented with or contain inositol and wherein the final concentration of inositol is between 1 mg/ml and 20 mg/ml.

2. Culture medium for use in a method according to claim 1, wherein said inositol is selected from: myo-inositol, D-chiro-inositol; L-chiro-inositol, scyllo-inositol, muco-inositol, neo-inositol, allo-inositol, epi-inositol and cis-inositol, or mixtures thereof.

3. Culture medium for use in a method according to claims 1 - 2 comprising: inositol, sodium chloride, potassium chloride, magnesium sulfate, potassium sulfate, calcium lactate, sodium bicarbonate, glucose, sodium pyruvate, sodium lactate, taurine, alanine - glutamine, EDTA, gentamicin, phenol red, human serum albumin, HEPES.

4. Culture medium for use in a method according to claims 1 - 3, wherein inositol is myo-inositol.

5. Culture medium for use in a method according to claims 1 - 4 further comprising methionine and glutathione.

6. Culture medium for use in a method according to claims 1 - 5 wherein said culture media contains:
| Component | Amount |
|---|---|
| Myo-inositol | 1-20 mg/ml |
| Sodium chloride | 5-7 mg/ml |
| Potassium chloride | 0.3-0.5 mg/ml |
| Magnesium sulfate | 0.02-0.03 mg/ml |
| Potassium sulfate | 0.002-0.01 mg/ml |
| Calcium lactate | 0.05-0.07 mg/ml |
| Sodium bicarbonate | 0.3-0.4 mg/ml |
| HEPES | 4.5-5.5 mg/ml |
| Glucose | 0.45-0.55 mg/ml |
| Sodium pyruvate | 0.2-0.4 mg/ml |
| Sodium lactate | 0.00045-0.00055 mM |
| Taurine | 0.008-0.01 mg/ml |
| Alanyl-Glutamine | 0.2-0.3 mg/ml |
| EDTA | 0.002-0.003 mg/ml |
| Gentamicin | 0.004-0.005 mg/ml |
| Phenol Red | 2.7-3.3 mg/L |
| Human serum albumin | 4.5-5.5 mg/L |

## Patentansprüche

1. Kulturmedien zur Verwendung bei der Behandlung von Eizellen und Embryonen in medizinisch betreuten Reproduktionstechniken, wobei die Kulturmedien mit Inositol angereichert oder Inositol enthalten und die finale Konzentration von Insositol zwischen 1 mg/ml und 20 mg/ml beträgt.

2. Kulturmedien zur Verwendung in einem Verfahren nach Anspruch 1, wobei das Inositol ausgewählt wird aus: Myo-Inositol, D-Chiro-Inositol, L-Chiro-Inositol, Scyllo-Inositol, Muco-Inositol, Neo-Inositol, Allo-Inositol, Epi-Inositol und CIS-Inositol oder einer Mischung davon.

3. Kulturmedien zur Verwendung in einem Verfahren nach Anspruch 1 bis 2, aufweisend: Inositol, Natriumchlorid, Kaliumchlorid, Magnesiumsulfat, Kaliumsulfat, Kalziumlaktat, Natriumbikarbonat, Glukose, Natriumpyruvat, Natriumlaktat, Taurin, Alanin-Glutamine, EDTA, Gentamicin, Phenolrot, humanes Serumalbumin, HEPES.

4. Kulturmedien zur Verwendung in einem Verfahren gemäß den Ansprüchen 1 bis 3, wobei das Inositol ein Myo-Inositol ist.

5. Kulturmedien zur Verwendung in einem Verfahren nach einem der Ansprüche 1 bis 4, weiter aufweisend Melatonin und Glutathion.

6. Kulturmedien zur Verwendung in einem Verfahren nach den Ansprüchen 1 bis 5, wobei die Kulturmedien aufweisen:
| Komponente | Menge |
|---|---|
| Myo-Inositol | 1-20 mg/ml |
| Natriumchlorid | 5-7 mg/ml |
| Kaliumchlorid | 0,3-0,5 mg/ml |
| Magnesiumsulfat | 0,02-0,03 mg/ml |
| Kaliumsulfat | 0,002-0,01 mg/ml |
| Kalziumlaktat | 0,05-0,07 mg/ml |
| Natriumbikarbonat | 0,3-0,4 mg/ml |
| HEPES | 4,5-5,5 mg/ml |
| Glukose | 0,45-0,55 mg/ml |
| Natriumpyruvat | 0,2-0,4 mg/ml |
| Natriumlaktat | 0,00045-0,00055 mM |
| Taurin | 0,008-0,01 mg/ml |
| Alanyl-Glutamin | 0,2-0,3 mg/ml |
| EDTA | 0,002.0,003 mg/ml |
| Gentamicin | 0,004-0,005 mg/ml |
| Phenolrot | 2,7-3,3 mg/L |
| Humanes Serumalbumin | 4,5-5,5 mg/L |

## Revendications

1. Milieux de culture destinés à être utilisés dans le traitement d'ovocytes et d'embryons dans des techniques de reproduction médicalement assistée, dans lesquels ces milieux de culture sont enrichis en ou contiennent de l'inositol et dans lesquels la concentration finale d'inositol est comprise entre 1 mg/ml et 20 mg/ml.

2. Milieu de culture destiné à être utilisé dans un procédé selon la revendication 1, dans lequel ledit inositol est choisi parmi : le myo-inositol, le D-chiro-inositol, le L-chiro-inositol, le scyllo-inositol, le muco-inositol, le néo-inositol, l'allo-inositol, l'épi-inositol et le cis-inositol, ou des mélanges de ceux-ci.

3. Milieu de culture destiné à être utilisé dans un procédé selon les revendications 1 et 2 comprenant : de l'inositol, du chlorure de sodium, du chlorure de potassium, du sulfate de magnésium, du sulfate de potassium, du lactate de calcium, du bicarbonate de sodium, du glucose, du pyruvate de sodium, du lactate de sodium, de la taurine, de l'alanine glutamine, de l'EDTA, de la gentamicine, du rouge phénol, de l'albumine sérique humaine, et de l'HEPES.

4. Milieu de culture destiné à être utilisé dans un procédé selon les revendications 1 à 3, dans lequel l'inositol est le myo-inositol.

5. Milieu de culture destiné à être utilisé dans un procédé selon les revendications 1 à 4, comprenant en outre de la mélatonine et du glutathion.

6. Milieu de culture destiné à être utilisé dans un procédé selon les revendications 1 à 5, dans lequel ledit milieu de culture contient :
| Composant | Quantité |
|---|---|
| Myo-inositol | 1 à 20 mg/ml |
| Chlorure de sodium | 5 à 7 mg/ml |
| Chlorure de potassium | 0,3 à 0,5 mg/ml |
| Sulfate de magnésium | 0,02 à 0,03 mg/ml |
| Sulfate de potassium | 0,002 à 0,01 mg/ml |
| Lactate de calcium | 0,05 à 0,07 mg/ml |
| Bicarbonate de sodium | 0,3 à 0,4 mg/ml |
| HEPES | 4,5 à 5,5 mg/ml |
| Glucose | 0,45 à 0,55 mg/ml |
| Pyruvate de sodium | 0,2 à 0,4 mg/ml |
| Lactate de sodium | 0,00045 à 0,00055 mg/ml |
| Taurine | 0,008 à 0,01 mg/ml |
| Alanyl-Glutamine | 0,2 à 0,3 mg/ml |
| EDTA | 0,002 à 0,003 mg/ml |
| Gentamicine | 0,004 à 0,005 mg/ml |
| Rouge de phénol | 2,7 à 3,3 mg/L |
| Albumine sérique humain | 4,5 à 5,5 mg/L |
